Europäisches Patentamt

(19) European Patent Office

Office européen des brevets

(11) Veröffentlichungsnummer: **0 201 611**
**A1**

## (12) EUROPÄISCHE PATENTANMELDUNG

(21) Anmeldenummer: 85105748.9

(22) Anmeldetag: 10.05.85

(51) Int. Cl.⁴: **A 61 M 5/32**

(43) Veröffentlichungstag der Anmeldung:
20.11.86 Patentblatt 86/47

(84) Benannte Vertragsstaaten:
AT BE CH DE FR GB IT LI LU NL SE

(71) Anmelder: INTERMEDICAT GMBH
Gerliswilstrasse 74
CH-6020 Emmenbrücke(CH)

(72) Erfinder: Bäumle, Hubert
Wanne
CH-6182 Escholzmatt(CH)

(72) Erfinder: Schwöbel, Eckhard
Reckenbühlstrasse 17
CH-6005 Luzern(CH)

(74) Vertreter: Maspoli, Renato A., Dipl.-Chem.Ing.ETH
Patentanwälte R.A. Maspoli und Partner
Promenadengasse 18
CH-8001 Zürich(CH)

(54) Zwei-Kanülen-Spritze.

(57) Die neue Zwei-Kanülen-Spritze zum Aspirieren und zum Spritzen von Medikamenten in Form von Flüssigkeiten weist eine erste Kanüle (01) zur Aspirierung der Flüssigkeit in den Spritzenzylinder und zur eventuellen Durchmischung und/oder Entgasung der Flüssigkeit und eine zweite Kanüle (02) zur eigentlichen Injektion auf. Beide Kanülen liegen geschützt vor, wobei die erste Kanüle versetzt gegenüber der zweiten und/oder in Längsrichtung der Spritze verschiebbar oder abnehmbar angeordnet ist.

Verwendet wird die neue Zwei-Kanülen-Spritze zur kontaminations-freien Injektion von Medikamenten in Form von Flüssigkeiten bei auswechslungsloser Benützung einer Zweitkanüle für die Injektion.

Fig. 3B

EP 0 201 611 A1

## ZWEI-KANUELEN-SPRITZE

Diese Erfindung betrifft eine Zwei-Kanülen-Spritze zum Aspirieren und Spritzen von Medikamenten in Form von Flüssigkeiten.

Injektionsspritzen mit abnehmbarer und daher auch auswechselbarer Kanüle sind bekannt, unter anderem aus den CH-Patenten 590 661 und 639 856 sowie aus der EP-Anmeldung, Ver. Nr. 0 047 442.

Spritzen für die Selbstinjizierung von Insulinlösungen werden unter anderem in der EP-Anmeldung, Ver. Nr. 0 045 367, beschrieben. Auf die bei derartigen Applikationen auftretenden Kontaminationsgefahren wird in dieser EP-Anmeldung zwar allgemein aufmerksam gemacht (Seite 3), dass es aber die Kanüle ist, die - vor allem bei mehrmaliger Benützung - die grösste Kontaminationswahrscheinlichkeit zeigt und somit die grösste Infektionsgefahr darstellt, wird nicht speziell erwähnt.

Auch das Arbeiten mit Wegwerf-Injektionsspritzen bringt dabei nur eine ungenügende Verringerung der Kontaminationsgefahr: Es ist die über längere Zeit benutzte Einstichstelle der Medikamentenflasche, die primär für die Uebertragung von Keimen auf die Kanüle in Frage kommt.

Ja selbst das Auswechseln der Kanüle zwischen dem Aspirieren und dem Injizieren kann noch zu Kontaminationen führen, vor allem wenn

2

die Lösung in der Spritze während des Wechsels nicht abgedeckt bleibt oder wenn das Auswechseln die Berührung der Kanüle bedingt.

Eine doppelkanülige Entnahmevorrichtung für sterile Anwendungen ist in der EP-Anmeldung, Ver. Nr. 0 085 957, beschrieben und beansprucht. Speziell in den Vorrichtungen gemäss Figuren 5 und 9 dieser Anmeldung wird eine einstechbare Doppelkanüle dargestellt, bei denen die beiden Kanülen aneinanderliegend ausgeführt sind und die auch miteinander in den Lösungsbehälter eingestochen werden. Durch die eine der Kanülen wird Flüssigkeit entnommen, durch die andere wird - zwecks Druckausgleichs - keimfrei Luft in den Behälter eingeleitet.

Das Arbeiten mit einer Kanüle für Aspirieren bzw. Aufziehen und für Injizieren birgt zudem die Gefahr der Beschädigung der Kanüle, was sich beim Spritzen negativ auswirken kann.

Erst durch die Zwei-Kanülen-Spritze gemäss der hier beschriebenen Erfindung wird aber grundsätzlich das Problem der auswechselfreien Kanülenänderung zwischen Aspiration und Injektion erkannt und gelöst.

Gemäss der Erfindung wird eine Zwei-Kanülen-Spritze zum Aspirieren und zum Spritzen von Medikamenten in Form von Flüssigkeiten geschaffen, die dadurch gekennzeichnet ist, dass die Spritze aufweist, a) eine erste Kanüle 01 zur Aspirierung der Flüssigkeit in den Spritzenzylinder und zur eventuellen Durchmischung und/oder Entgasung der Flüssigkeit und b) eine zweite Kanüle 02 zur eigentlichen Injektion, wobei beide Kanülen geschützt vorliegen, wobei die erste Kanüle 01 gegenüber der zweiten versetzt und/oder entfernbar oder in Längsrichtung der Spritze verschiebbar angeordnet ist und wobei beide Kanülen um die gleiche Spritzen-Längsachse angeordnet sind.

Eine Gruppe von erfindungsgemässen Zwei-Kanülen-Spritzen ist so ausgebildet, dass die erste Kanüle 01 entfernbar vor der zweiten Kanüle 02 angeordnet ist, wobei um die erste Kanüle eine Schutzkappe 03 mit Filter zur Umgebung 04 liegt und die Halterung 05 der genannten ersten Kanüle die Schutzkappe der genannten zweiten Kanüle bildet und wobei die zweite Kanüle während der Lagerungs- und Aspirationsphasen in die erste Kanüle hineinragt.

Eine zweite Gruppe von erfindungsgemässen Zwei-Kanülen-Spritzen ist so ausgebildet, dass die erste Kanüle 01 entfernbar vor der zweiten Kanüle 02 angeordnet ist, wobei um die erste Kanüle eine Schutzkappe 03 liegt und die Halterung 05 der genannten ersten Kanüle die Schutzkappe der genannten zweiten Kanüle mit Einschluss eines Zwischenfilters 04 bildet und wobei die zweite Kanüle während der Lagerungs- und Aspirationsphase hinter der ersten Kanüle liegt und nicht in diese hineinragt.

Eine weitere Gruppe von erfindungsgemässen Zwei-Kanülen-Spritzen ist so ausgebildet, dass die erste Kanüle 01 in einer hinsichtlich der Spritze beweglichen Halterung 05' befestigt ist, welche Halterung durch Drehung des äusseren Führungsringes 06 um den Schraubengang 07 in der Längsrichtung vor- und rückwärts bewegbar ist, wodurch die erste Kanüle vor bzw. hinter die Spitze der zweiten Kanüle verlegt wird, wobei sich erste und zweite Kanülen während der Lagerungsphase in einer Schutzkappe 03 mit Filter zur Umgebung 04 befinden.

Schliesslich ist eine letzte Gruppe von erfindungsgemässen Zwei-Kanülen-Spritzen so ausgebildet, dass die erste Kanüle 01 in einer hinsichtlich der Spritze beweglichen Halterung 05" befestigt ist, welche Halterung durch Schieben in einer Aussparung des Führungszylinders 08 in der Längsrichtung vor- und rückwärts bewegbar ist, wodurch die erste Kanüle vor, bzw. hinter die Spitze der zweiten Kanüle verlegt wird, wobei erste und zweite Kanülen während der Lagerungsphase sich in einer Schutzkappe mit Filter zur Umgebung befinden.

Bei den letzten beiden Gruppen von Zwei-Kanülen-Spritzen können am Anfang und am Ende der Bewegungsstrecke der Halterung (05', 05") Fixierungen für dieselbe vorgesehen sein.

Die beschriebenen Zwei-Kanülen-Spritzen werden aus den dafür geeigneten Materialien, insbesondere aus Kunststoffen für Spritzenkörper, Zylinder, Kolbenstange und Schutzkappe, Metall oder Kunststoff, speziell Stahl für die Kanüle und Synthese- oder Naturkautschuk für Kolben und mittels kostengünstiger Methoden hergestellt und sterilisiert.

Verwendung finden die erfindungsgemässen Zwei-Kanülen-Spritzen zur Aspirierung und Injizierung von Medikamenten in Form von Flüssigkeiten ohne Auswechseln der Kanüle, wozu Lagerungs- bzw. Transportphasen, Aspirationsphasen und Injektionsphasen zu unterscheiden sind.

Speziell werden die Zwei-Kanülen-Spritzen gemäss der obigen ersten und zweiten Gruppe so verwendet, dass während der Lagerungs- bzw. Transportphase die erste Kanüle vor der zweiten Kanüle angeordnet ist, dass beide Kanülen von einer Schutzkappe geschüzt sind, dass zur Aspirierung bzw. Mischung/Entgasung der Flüssigkeit die Schutzkappe 03 entfernt wird und dass zur Injektion auch die erste Kanüle samt vollständiger Halterung 05 entfernt wird.

Speziell werden die Zwei-Kanülen-Spritzen gemäss der obigen weiteren und letzten Gruppe so verwendet, dass während der Lagerungs- bzw. Transportphase die zweite Kanüle jedenfalls und die erste Kanüle gegebenenfalls von einer Schutzkappe geschützt sind, dass zur Aspirierung bzw. Mischung/Entgasung der Flüssigkeit die Schutzkappe 03 entfernt und die erste Kanüle in die Stellung vor der Spitze der zweiten Kanüle gebracht und dass zur Injektion die erste Kanüle in die Stellung hinter die Spitze der zweiten Kanüle gebracht wird.

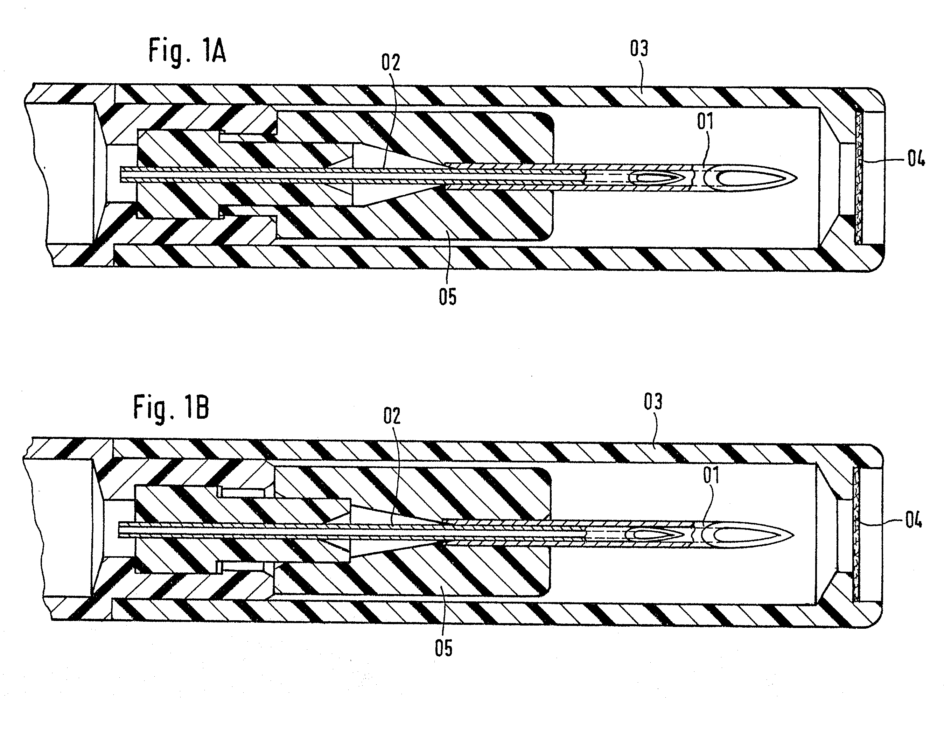

Im folgenden werden die oben beschriebenen Gruppen der Ausführungsformen der erfindungsgemässen Zwei-Kanülen-Spritzen anhand der beiliegenden Darstellungen in Figuren 1 bis 4 genauer erläutert; zugleich werden auch die oben schon verwendeten Hinweisungszahlen illustriert.

Die Figuren 1A und 1B zeigen die erfindungsgemässen Zwei-Kanülen-Spritzen der oben beschriebenen ersten Gruppe in der Lagerungs- und Transportphase. Die zweite Kanüle 02 liegt dabei in der ersten 01; beide sind in der Kappe 03 mit Filter gegen aussen 04 versorgt. Zur Aspirierung wird die Schutzkappe entfernt. Nach dem Füllen des Spritzenzylinders mit der Flüssigkeit wird die erste Kanüle mit Halterung 05 entfernt. Dadurch erst wird die zweite Kanüle frei für die eigentliche Injektion.

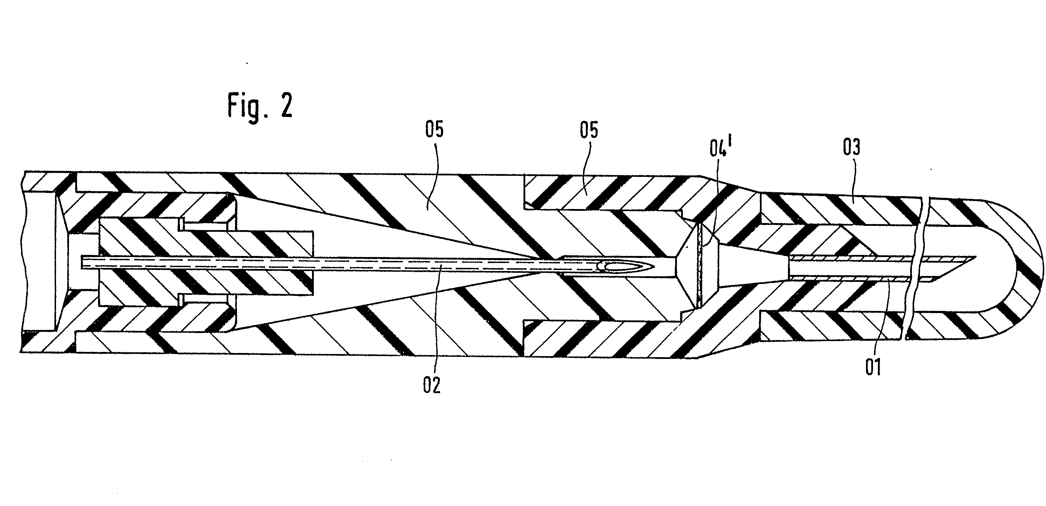

Die Figur 2 zeigt die erfindungsgemässe Zwei-Kanülen-Spritze der oben beschriebenen zweiten Gruppe, ebenfalls in der Lagerungs- und Transportphase. Die erste Kanüle 02 liegt getrennt vor der zweiten Kanüle 02. Um die erste Kanüle liegt die Schutzkappe 03, die zwecks Aspiration entfernt wird. Beim Aufsaugen der Flüssigkeit passiert diese das Filter 04', welches aus einem dafür geeigneten Material hergestellt ist und in der Halterung 05 der ersten Kanüle befestigt ist. Nach der Aspiration wird die erste Kanüle mit ihrer Halterung und mit dem Zwischenfilter entfernt; Kanüle 02 ist für die Injektion frei.

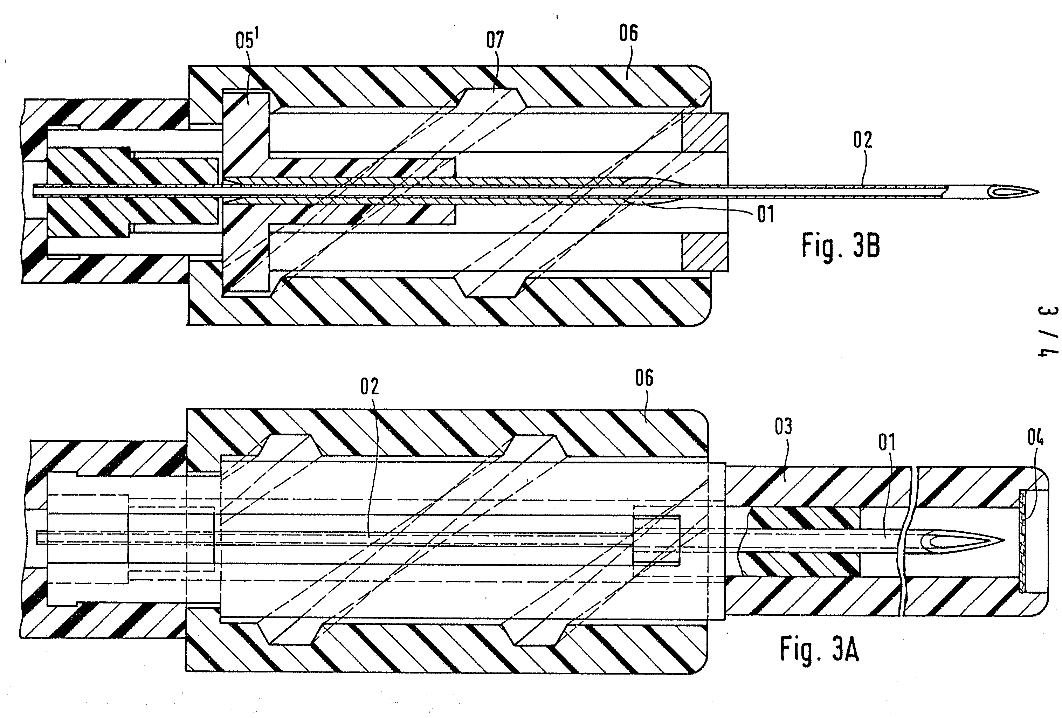

Die Figuren 3A und 3B zeigen die erfindungsgemässen Zwei-Kanülen-Spritzen der oben beschriebenen weiteren Gruppe und in der Lagerungs- bzw. Transportphase (3A) und in der Injektionsphase (3B).

In Figur 3A sind zu erkennen: Die erste Kanüle 01, die zweite Kanüle 02, die Schutzkappe 03 und der Führungsring 06.

In Figur 3B erkennt man zusätzlich zu den eben genannten Bauteilen der erfindungsgemässen Zwei-Kanülen-Spritzen die eigentliche Halterung 05' der ersten Kanüle und den Schraubengang 07.

Zur Aspirierung von Flüssigkeit wird die Schutzkappe 03 entfernt und die erste Kanüle in die Stellung gemäss Figur 3A gebracht, bzw. dort belassen. Nach dem Aufsaugen der Flüssigkeit in die Spritze wird die erste Kanüle 01 durch Drehung des Führungsringes 06 in die Stellung gemäss Figur 3B gebracht; die Spritze ist für die Injektion bereit.

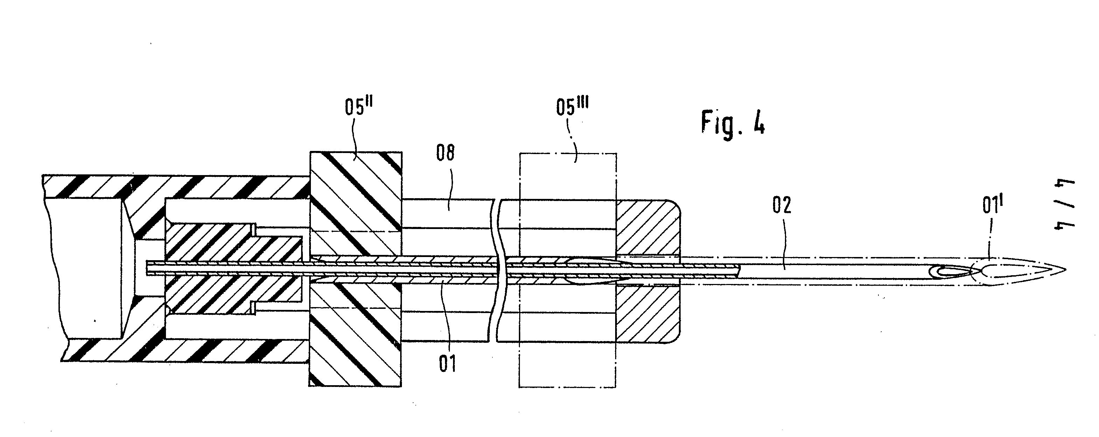

Die Figur 4 schliesslich zeigt die erfindungsgemässe Zwei-Kanülen-Spritze der oben beschriebenen letzteren Gruppe der erfindungsgemässen Ausführungsformen ohne Schutzkappe in verschiedenen Verwendungsphasen.

Während der Aspiration befindet sich die erste Kanüle mit Halterung in Stellung vorn 01', 05"', anschliessend, d.h. bei der Injektion in Stellung hinten 01, 05". Die Führung der genannten Halterung geschieht durch Verschieben derselben in einer oder mehreren Aussparungen im Führungszylinder 08.

Dass bei der erfindungsgemässen Zwei-Kanülen-Spritze während der Lagerungs- und Aufziehphasen die eigentliche Injektionskanüle vor Beschädigungen geschützt ist, ist hier ebenfalls anzumerken.

Die beschriebenen vier Gruppen der erfindungsgemässen Zwei-Kanülen-Spritzen sind als beispielshafte Angaben anzusehen; ohne weiteres sind aufgrund des Erfindungskonzeptes weitere Ausführungsformen möglich.

PATENTANSPRUECHE

1.

Zwei-Kanülen-Spritze zum Aspirieren und zum Spritzen von Medikamenten in Form von Flüssigkeiten, dadurch gekennzeichnet, dass die Spritze aufweist

a) eine erste Kanüle (01) zur Aspirierung der Flüssigkeit in den Spritzenzylinder und zur eventuellen Durchmischung und/oder Entgasung der Flüssigkeit und

b) eine zweite Kanüle (02) zur eigentlichen Injektion,

wobei beide Kanülen geschützt vorliegen, wobei die erste Kanüle (01) gegenüber der zweiten versetzt und/oder entfernbar oder in Längsrichtung der Spritze verschiebbar angeordnet ist und wobei beide Kanülen um die gleiche Spritzen-Längsachse angeordnet sind.

2.

Zwei-Kanülen-Spritze gemäss Patentanspruch 1, dadurch gekennzeichnet, dass die erste Kanüle (01) entfernbar vor der zweiten Kanüle (02) angeordnet ist, wobei um die erste Kanüle (01) eine Schutzkappe (03) mit Filter zur Umgebung (04) liegt und die Halterung (05) der genannten ersten Kanüle die Schutzkappe der genannten zweiten Kanüle bildet und wobei die zweite Kanüle während der Lagerungs- und Aspirationsphasen in die erste Kanüle hineinragt.

3.

Zwei-Kanülen-Spritze gemäss Patentanspruch 1, dadurch gekennzeichnet, dass die erste Kanüle (01) entfernbar vor der zweiten Kanüle (02) angeordnet ist, wobei um die erste Kanüle (01) eine Schutzkappe (03) liegt und die Halterung (05) der genannten ersten Kanüle die Schutzkappe der zweiten Kanüle mit Einschluss eines Zwischenfilters (04') bildet und wobei die zweite Kanüle während der Lagerungs-und Aspirationsphase hinter der ersten Kanüle liegt und nicht in diese hineinragt.

4.

Zwei-Kanülen-Spritze gemäss Patentanspruch 1, dadurch gekennzeichnet, dass die erste Kanüle (01) in einer hinsichtlich der Spritze beweglichen Halterung (05') befestigt ist, welche Halterung durch Drehung des äusseren Führungsringes (06) um den Schraubengang (07) in der Längsrichtung vor- und rückwärts bewegbar ist, wodurch die erste Kanüle vor bzw. hinter die Spitze der zweiten Kanüle (02) verlegt wird, wobei sich erste und zweite Kanülen während der Lagerungsphase in einer Schutzkappe (03) mit Filter zur Umgebung (04) befinden.

5.

Zwei-Kanülen-Spritze gemäss Patentanspruch 1, dadurch gekennzeichnet, dass die erste Kanüle (01) in einer hinsichtlich der Spritze beweglichen Halterung (05") befestigt ist, welche Halterung durch Schieben in einer Aussparung des Führungszylinders (08) in der Längsrichtung vor- und rückwärts bewegbar ist, wodurch die erste Kanüle vor bzw. hinter die Spitze der zweiten Kanüle verlegt wird, wobei erste und zweite Kanülen während der Lagerungsphase sich in einer Schutzkappe mit Filter zur Umgebung befinden.

6.

Zwei-Kanülen-Spritze gemäss Patentansprüchen 4 und 5, dadurch gekennzeichnet, dass am Anfang und am Ende der Bewegungsstrecke der Halterung (05', 05") Fixierungen für dieselbe vorgesehen sind.

7.

Zwei-Kanülen-Spritze gemäss allen vorangehenden Patentansprüchen, dadurch gekennzeichnet, dass die Spritze aus den dafür geeigneten Materialien, insbesondere aus Kunststoffen für Spritzenkörper, Zylinder, Kolbenstange und Schutzkappe, Metall oder Kunststoff, speziell Stahl für die Kanüle und Synthese- oder Naturkautschuk für Kolben, und dass sie mittels kostengünstiger Methoden hergestellt und sterilisiert wird.

8.

Verwendung der Zwei-Kanülen-Spritze gemäss Patentanspruch 1 zur Aspirierung und Injizierung von Medikamenten in Form von Flüssigkeiten ohne Auswechseln der Kanüle, dadurch gekennzeichnet, dass dazu die Lagerungs- bzw. Transportphase, die Aspirationsphase und die Injektionsphase unterschieden werden.

9.

Verwendung gemäss Patentanspruch 8 der Zwei-Kanülen-Spritzen gemäss Patentansprüchen 2 und 3, dadurch gekennzeichnet, dass

während der Lagerungs- bzw. Transportphase die erste Kanüle (01) vor der zweiten Kanüle (02) angeordnet ist und dass beide Kanülen von je einer Schutzkappe (03) geschützt sind, dass

zur Aspirierung bzw. Mischung/Entgasung der Flüssigkeit die Schutzkappe (03) entfernt wird und dass

zur Injektion auch die erste Kanüle (01) samt vollständiger Halterung (05) entfernt wird.

10.

Verwendung gemäss Patentanspruch 8 der Zwei-Kanülen-Spritzen gemäss Patentansprüchen 4 und 5, dadurch gekennzeichnet, dass

während der Lagerungs- bzw. Transportphase die zweite Kanüle (02) jedenfalls und die erste Kanüle (01) gegebenenfalls von einer

Schutzkappe geschützt sind, dass

zur Aspirierung bzw. Mischung/Entgasung der Flüssigkeit die Schutzkappe (03) entfernt und die erste Kanüle (01) in die Stellung vor der Spitze der zweiten Kanüle (02) gebracht wird und dass

zur Injektion die erste Kanüle in die Stellung hinter die Spitze der zweiten Kanüle gebracht wird.

Fig. 1A

Fig. 1B

Fig. 2

05'    07    06

01

02

Fig. 3B

02    06

03    01    04

Fig. 3A

Fig. 4

0201611

**Europäisches Patentamt**

## EUROPÄISCHER RECHERCHENBERICHT

Nummer der Anmeldung

EP 85 10 5748

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | DE-C- 378 629 (H. KOCH) * Anspruch; Figuren * | 1,5,6 | A 61 M 5/32 |
| X | DE-U-1 678 482 (L.H. ELLINGHAUSEN) * Ansprüche 1-3; Figuren 1-3 * | 1,5,8, 10 | |
| A | DE-U-7 310 426 (VYGON ERZEUGNISSE FÜR MEDIZIN UND CHIRURGIE GMBH & CO. KG) * Anspruch 1; Figuren 1-3 * | 1,7 | |
| A | DE-A-2 554 333 (C.A. HENRIQUEZ DE CAZTANONDO) * Anspruch 1; Figuren 1-3, 6 * | 1 | |
| A | DE-A-2 507 119 (K. SOKOL) * Anspruch 1; Figuren 1-4, 8 * | 1,2 | RECHERCHIERTE SACHGEBIETE (Int. Cl.4) |
| A | US-A-3 081 770 (J.M. HUNTER) * Spalte 3, Zeilen 3-41; Figuren 1-4 * | 1 | A 61 M 5/00 |
| A | FR-A-2 366 026 (S. OIWA) * Anspruch 1; Figuren 1-5 * | 2,3 | |

Der vorliegende Recherchenbericht wurde für alle Patentansprüche erstellt.

| Recherchenort BERLIN | Abschlußdatum der Recherche 20-12-1985 | Prüfer MASSALSKI W. |
|---|---|---|